# EUROPEAN PATENT APPLICATION

(11) **EP 1 872 777 A1**
(43) Date of publication of application: **02.01.2008**
(21) Application number: 06013188.5
(22) Date of filing: 27.06.2006
(51) Int. Cl.: A61K 9/16, A61K 31/365

(54) **Pharmaceutical composition comprising tetrahydrolipstatin**

(71) Applicant: LEK Pharmaceuticals d.d., 1526 Ljubljana (SI)
(72) Inventor: The designation of the inventor has not yet been filed
(74) Representative: Dietz, Jörg-Reimar

(57) **Abstract**

The present invention relates to a stable pharmaceutical composition comprising tetrahydrolipstatin, carrageenan and other pharmaceutically acceptable excipients, characterized in that it is in the form of multiple unit solid dosage form with improved stability characteristics.

## Description

### Field of the invention

The present invention belongs to the field of pharmaceutical technology and relates to a novel pharmaceutical composition comprising tetrahydrolipstatin. Tetrahydrolipstatin is an active pharmaceutical substance also known as orlistat. Orlistat reduces the absorbtion of dietary fat and in such a manner prevents and treats obesity. The present invention relates to a stable pharmaceutical composition comprising tetrahydrolipstatin, carrageenan and other pharmaceutically acceptable excipients, characterized in that it is in the form of multiple unit dosage form with improved stability characteristics.

### Background of the invention

Tetrahydrolipstatin is an inhibitor of pancreatic lipase and is used for the control or prevention of obesity and hyperlipaemia. Chemically, it is N-formyl-L-leucine[2S-[2alpha(R*),3beta]]-1-[[3-hexyl-4-oxo-2-oxetanyl]methyl]dodecyl ester and is known by the generic name orlistat. The use of tetrahydrolipstatin as medicament and pharmaceutical compositions containing tetrahydrolipstatin as active agent are described in US Patent No. 4 598 089.

In European patent EP 0 921 796 B1 is disclosed that tetrahydrolipstatin is susceptible to hydrolysis and thermal degradation, particularly when stored in a humid atmosphere or above 35°C in a dry atmosphere.

Tetrahydrolipstatin is marketed by Hoffmann-La Roche with the brand name Xenical^{®}. According to the originator's guidance written on the packaging the storage in a dry place below 25°C in blister packs or below 30°C when stored in glass bottles with desiccant is prescribed.

Furthermore, the European patent EP 0 921 796 B1 disclose that the formulating of a dosage forms containing tetrahydrolipstatin from a powder mix or by conventional wet granulation procedure is problematic due to picking and sticking phenomena during tablet compression or encapsulation.

EP 0 921 796 B1 discloses pharmaceutical compositions containing tetrahydrolipstatin as active substance, stabilizers and pharmaceutically acceptable excipients, characterized in that they are preferably in the form of or pellets. The preferred pellet form requires the presence of microcrystalline cellulose and does not feature the sticking and picking phenomena but that the product disclosed in said patent exhibits superior tetrahydrolipstatin stability.

In Physicians' desk reference for Xenical® capsules is disclosed that tetrahydrolipstatin is practically insoluble substance. Because of a long disintegration time of tetrahydrolipstatin, pharmaceutical composition comprising tetrahydrolipstatin would additionally decrease the dissolution rate of tetrahydrolipstatin. Therefore fast disintegrating pharmaceutical composition is desired. Pellets containing microcrystalline cellulose do not disintegrate, as it is said in International Journal of Pharmaceutics, volume 109 (1994), p. 221-227, that results in slow drug release, especially for low soluble drug substances, as it is disclosed in European Journal of Pharmaceutics and Biopharmaceutics, volume 56 (2003), p. 371-380 and European Journal of Pharmaceutics and Biopharmaceutics, volume 59 (2005), p. 127-131. This undesired property of pellets can be only partially overcome by addition of soluble fillers, disintegrants and high amounts of surface active substances. Thus, the production of fast release pellets by extrusion/spheronization method using microcrystalline cellulose is difficult. Additionally, it might be expected that high amounts of surface active substances might negatively affect the stability of tetrahydrolipstatin, by means of increasing its wetability and consequently increasing the degree of its hydrolytic degradation.

Carrageenan is known from the literature as new pelletization aid which can be used as an alternative to microcrystalline cellulose in extrusion/spheronization process. The use of carrageenan as a pelletization aid was introduced in European Journal of Pharmaceutics and Biopharmaceutics, volume 59, 2005, p. 127-131. In European Journal of Pharmaceutics and Biopharmaceutics, volume 63, 2006, p. 59-67 and volume 63, 2006, p. 68-75 is disclosed that high quality pellets that own fast disintegration and fast drug release characteristics can be prepared with K-carrageenan.

In above mentioned literature different pharmaceutical substances were used for pellet preparation using carrageenan, but tetrahydrolipstatin was not mentioned.

In European Journal of Pharmaceutics and Biopharmaceutics volume 59, 2005, p. 127-131 is disclosed that K-carrageenan requires higher water content compared to microcrystalline cellulose to produce spherical granules. This was recognized as a disadvantage of K-carrageenan over microcrystalline cellulose, because of long drying process and possible stability problem for sensitive drugs.

As can be concluded, no literature discloses in a satisfactory manner solving the problem of poor stability of the pharmaceutical formulation with tetrahydrolipstatin. No literature discloses neither solving the problem of the stability of any other drug substance by using carrageenan. On the contrary, it was presumed that K-carrageenan could negatively affect the stability of drug substances sensitive to presence of water and heating during long drying process.

As it is desired for the drug product to contain as less as possible degradation products there is still a need for developing a stable pharmaceutical composition comprising tetrahydrolipstatin, where excellent stability against moisture and heat during production and storage is achieved.

### Summary of the invention

The subject of the invention is a pharmaceutical composition comprising tetrahydrolipstatin, carrageenan and at least one pharmaceutically acceptable excipient, characterized in that the pharmaceutical composition is in the form of a multiple unit dosage form, wherein said composition comprises about 2 to 80% of carrageenan.

Pharmaceutical composition according to the present invention could also contain pharmaceutical acceptable excipient which is selected from a group of fillers, surfactants, and binders.

Pharmaceutical composition according to the present invention show excellent results during the stability testing.

Another subject of the invention is the composition comprising tetrahydrolipstatin characterized in that the increase of the total degradation products in the composition is not more than about 0.5% when stored at accelerated storage condition of 40 °C and 75 % relative humidity for 14 days.

Another subject of the invention is a use of the composition according to the present invention in the manufacture of a medicament useful for the treatment of obesity and hyperlipaemia.

Another subject of the invention is a use of the composition according to the present invention in a medicament useful for the treatment of obesity and hyperlipaemia.

### Detailed description of the invention

The pharmaceutical composition according to the present invention comprises tetrahydrolipstatin as an active substance, carrageenan and other pharmaceutically acceptable excipients, characterized in that it is in the form of multiple unit dosage forms, such as granules, micro tablets and pellets, preferably pellets.

It was surprisingly found that pharmaceutical composition according to the present invention comprising tetrahydrolipstatin not only enables production of fast disintegrating particles and avoiding of sticking and picking phenomena during manufacture, but also exhibits significantly improved stability compared to the stability of the pure tetrahydrolipstatin substance itself. It was therefore found that carrageenan also acts as stabilizer which was not known up to know.

As it is disclosed in the literature above tetrahydrolipstatin is practically insoluble substance. Since long disintegration time of tetrahydrolipstatin comprising pharmaceutical composition additionally decreases the dissolution rate of tetrahydrolipstatin, fast disintegrating composition is desired. To form a useful pharmaceutical composition we select carrageenan due to its property to form disintegrating pellets in order to obtain disintegrating pellets comprising tetrahydrolipstatin by extrusion/spheronization method and assure fast enough release of tetrahydrolipstatin.

The pharmaceutical composition according to the present invention was prepared by the extrusion/spheronization method, which is well known from the art. Literature that discloses said method is, for example Encyclopedia of Pharmaceutical Technology, Marcel Dekker Inc. (1988), Vol. 11: p. 369-393 and International Journal of Pharmaceutics 116 (1995) p. 131-136.

The pharmaceutical composition according to the present invention was prepared by the extrusion/spheronization method comprising following operations:
1. dry mixing of tetrahydrolipstatin, carrageenan and other pharmaceutically acceptable excipients, including also the sieving of the powder mixture,
2. optionally dissolving of the binder and/or the surface active substance and/or plasticizer in demineralized water,
3. granulating of the sieved powder mixture with demineralized water or optionally with the solution of the binder and/or the surface active substance and/or plasticizer in demineralized water - obtaining the wet mass for extrusion,
4. extruding of obtained wet mass for extrusion by extruder - obtaining the extrudate,
5. spheronizing of the obtained extrudate by spheronizer - obtaining the wet pellets,
6. drying of the wet pellets,
7. sieving of the dried pellets - obtaining selected fraction of the pellets.

Also other known methods from the art could be used for the preparation of the formulation of the present invention. Such methods are high shear granulation or pelletization, direct rotor pelletization, fluid bed granulation or tableting.

The pharmaceutical composition according to the present invention comprises 10 to 90% of tetrahydrolipstatin by weight, preferably 40 to 60, more preferably about 50%.

The pharmaceutical composition according to the present invention comprises 2 to 80% carrageenan, preferably 10 to 30%, more preferably 20%.

The pharmaceutical composition according to the present invention might also comprise up to 80% fillers, preferably 20 to 30%, such fillers are selected from a group of lactose, starch, sucrose, glucose, mannitol, sorbitol, powdered cellulose, dicalcium phosphate dihydrate, calcium sulphate, barium sulphate, and other pharmaceutically acceptable fillers, preferably lactose.

The pharmaceutical composition according to the present invention might optionally comprise up to 1% surface active substances, such as polysorbate, sodium lauryl sulphate, preferably sodium lauryl sulphate.

The pharmaceutical composition according to the present invention could also contain binders, such as polyvinylpyrrolidone, cellulose derivatives and dextrin, as well as enteric polymers that are soluble at higher pH values as for example higher than about pH 5.0-5.5 such as shellac, copolymers of methacrylic acid and esters of hydroxyalkylcellulose, such as hydroxypropyl methylcellulose phthalate and hydroxypropyl methylcellulose acetate succinate. These polymers might be useful for attaining dissolution rate that is in all physiological pH values comparable to the dissolution rate of the formulation containing tetrahydrolipstatin present on the market, namely Xenical^{®} (originator Hoffmann-La Roche).

The pharmaceutical composition according to the present invention could also contain plasticizers, such as for example polyethylene glycols of different molecular weight, cetyl alcohol, olive oil, castor oil, monoglycerides, diethyl phthalate, triethyl citrate, dibutyl sebacate, preferably triethyl citrate.

The pharmaceutical composition according to the present invention might optionally contain disinegrants, such as crosspovidone, sodium starch glycolate, crosscarmellose sodium.

The pharmaceutical composition according to the present invention can be filled into sachettes or capsules, preferably hard capsules. The stable pharmaceutical composition according to the present invention can also be compacted into tablets together with pharmaceutically acceptable excipients such as tablet fillers, binders, disintegrating agents, glidants, lubricants etc.

Pharmaceutical composition according to the present invention surprisingly showed excellent stability when stored at temperature above 35°C in a humid or in a dry atmosphere. A stability of the sample according to the present invention means an increase of total degradation products in percentage during 14 days, stored at 40 ± 2 °C and 75 ± 5 % relative humidity. Said storage condition was selected according to the U.S. Food and Drug Administration, Center for drug evaluation and research - ICH guideline standards for stability testing.

Stability was measured for the pharmaceutical composition according to the present invention (noted as Example 1), the pharmaceutical formulation according to EP 0 921 796 B1 (noted as Example 2) and pharmaceutical formulation containing tetrahydrolipstatin and is present on the market with commercial name Xenical^{®} (originator Hoffmann-La Roche). The increase of total degradation products was also measured for tetrahydrolipstatin substance itself. Results are presented in the table1.

**Table 1: Increase of total degradation products (%)**

| | | |
|---|---|---|
| Storage condition | 40 ± 2°C / 75 ± 5 % relative humidity | |
| Container closure | sealed glass vials | open dish |
| Time | 14 days | 14 days |

| | Increase of total degradation products - % | |
|---|---|---|
| Tetrahydrolipstatin substance | 0.01 % | 0.09 % |
| Example 1 | 0 | 0 |
| Example 2 | 0.64 % | 1.03 % |
| Xenical^{®} | 0.48 % | 2.40 % |

The stability results show that there is no significant difference in stability of the pharmaceutical formulation prepared according to EP 0 921 796 B1 and pharmaceutical formulation present on the market that contains tetrahydrolipstatin, namely Xenical^{®}. In both formulations high increase of total degradation products was observed when exposed to elevated temperature in sealed glass vials or elevated temperature and high humidity in open dish study. On the contrary, no increase of total degradation products was observed in the pharmaceutical composition according to the present invention when exposed to the same accelerated storage conditions.

The following Examples illustrate the present invention in more detail, but are not intended to limit its extent.

### Example 1

| Composition according to the present invention (in % w/w) | |
|---|---|
| Tetrahydrolipstatin | 50 |
| Carrageenan | 20 |
| Lactose | 29 |
| Sodium lauryl sulphate | 1 |
| Total | 100 |

### Preparation of pellets

The pellets were prepared in following steps:
1. dry mixing of tetrahydrolipstatin, carrageenan, lactose and sodium lauryl sulphate,
2. sieving of the powder mixture,
3. granulating of the sieved powder mixture with demineralized water - obtaining wet mass for extrusion,
4. extruding of obtained wet mass for extrusion by extruder - obtaining the extrudate,
**5.** spheronizing of the obtained extrudate by spheronizer - obtaining the wet pellets,
6. drying of the wet pellets,
7. sieving of the dried pellets - obtaining selected fraction of the pellets.

### Example 2

| Composition according to example 2 in EP 0 921 796 B1 (in % w/w) | |
|---|---|
| Tetrahydrolipstatin | 50 |
| Microcrystalline cellulose | 39 |
| Sodium starch glycolate | 3 |
| Sodium lauryl sulphate | 3 |
| Povidone K 30 | 5 |
| Total | 100 |

### Preparation of pellets

The pellets were prepared in following steps:
1. dissolving of sodium lauryl sulphate and povidone K 30 in demineralized water by a stirrer - obtaining the solution A,
2. dry mixing of tetrahydrolipstatin, microcrystalline cellulose and sodium starch glycolate.
3. sieving of the powder mixture,
4. granulating of the sieved powder mixture with solution A - obtaining wet mass for extrusion,
5. extruding of obtained wet mass for extrusion by extruder - obtaining the extrudate,
6. spheronizing of the obtained extrudate by spheronizer - obtaining the wet pellets,
7. drying of the wet pellets,
8. sieving of the dried pellets - obtaining selected fraction of the pellets.

### Example 3

Stability tests

To evaluate stability of pharmaceutical composition according to the present invention (noted as Example 1), the pharmaceutical composition prepared according EP 0 921 796 B1 (noted as Example 2) and pharmaceutical formulation present on the market that contains tetrahydrolipstatin, namely Xenical^{®} samples were exposed to 40 ± 2 °C and 75 ± 5 % relative humidity in a sealed glass vials and open dish for 14 days. After that increase of total degradation products was determinated by HPLC method. The same tests were performed with the tetrahydrolipstatin substance itself.

The increase of total degradation products was measured as the difference between the amount of total impurities after exposure to specific storage conditions and the amount of total impurities before exposure. The amount of total impurities in samples (given as a mass percentage relative to the tetrahydrolipstatin) was determined by the generally known HPLC method which is capable of resolving the tetrahydrolipstatin from the impurities in it.

Results of measured of total degradation products are presented in the Table 2 below and demonstrate the following:
- significant increase of total degradation products was observed for pharmaceutical composition prepared according to EP 0 921 796 B1 (noted as Example 2) and pharmaceutical formulation present on the market that contains tetrahydrolipstatin, namely Xenical^{®} at accelerated storage conditions after 14 days;
- no increase of total degradation products was detected for pharmaceutical composition according to the present invention (Example 1) when exposed to the same accelerated storage conditions for 14 days;
- the stability of pharmaceutical composition according to the present invention (Example 1) is improved when compared to the stability of the active substance itself.

**Table 2**

| | | |
|---|---|---|
| Storage condition | 40 ± 2 °C / 75 ± 5 % relative humidity | |
| Container closure | sealed glass vials open dish | |
| Time | 14 days 14 days | |

| | Increase of total degradation products - % | |
|---|---|---|
| Tetrahydrolipstatin substance | 0.01 % | 0.09 % |
| Example 1 | 0 | 0 |
| Example 2 | 0.64 % | 1.03 % |
| Xenical^{®} | 0.48 % | 2.40 % |

## Claims

1. A pharmaceutical composition comprising tetrahydrolipstatin, carrageenan and at least one pharmaceutically acceptable excipient, **characterized in that** the pharmaceutical composition is in the form of a multiple unit dosage form wherein said composition comprises about 2 to 80 % of carrageenan.

2. The composition according to the claim 1 wherein pharmaceutical acceptable excipient is selected from a group of fillers, surfactants, and binders.

3. The composition according to claim 1 wherein said composition comprises about 10 to 90% of tetrahydrolipstatin.

4. The composition according to claim 1 wherein said composition comprises about 40 to 60% of tetrahydrolipstatin.

5. The composition according to claim 1 wherein said composition comprises about 10 to 30% of carrageenan.

6. A composition comprising tetrahydrolipstatin **characterized in that** the increase of the total degradation products in the composition is not more than about 0.5% when stored at accelerated storage condition of 40 °C and 75 % relative humidity for 14 days.

7. The composition according to claim 6 **characterized in that** the increase of the total degradation products in the composition is not more than about 0.25% when stored at accelerated storage condition of 40 °C and 75 % relative humidity for 14 days.

8. The composition according to claim 6 **characterized in that** the increase of the total degradation products in the composition is not more than about 0.1% when stored at accelerated storage condition of 40 °C and 75 % relative humidity for 14 days.

9. The use of the composition according to any of previous claim in the manufacture of a medicament useful for the treatment of obesity and hyperlipaemia

10. The use of the composition according to any of claims 1 to 9 in a treatment of obesity and hyperlipaemia.
